(19) Europäisches Patentamt
European Patent Office
Office européen des brevets

(11) Publication number: **0 268 343 B1**

(12) **EUROPEAN PATENT SPECIFICATION**

(45) Date of publication of patent specification: **27.05.92**

(51) Int. Cl.[5]: **C12P 35/00**, C07D 501/18

(21) Application number: **87301255.3**

(22) Date of filing: **13.02.87**

(54) **Enzymatic process for 3-substituted cephalosporins.**

(30) Priority: **17.11.86 US 931102**

(43) Date of publication of application:
**25.05.88 Bulletin 88/21**

(45) Publication of the grant of the patent:
**27.05.92 Bulletin 92/22**

(84) Designated Contracting States:
**AT BE CH DE ES FR GB GR IT LI NL SE**

(56) References cited:
**EP-A- 0 102 216**
**US-A- 4 178 210**
**US-A- 4 307 192**

**JOURNAL OF THE CHEMICAL SOCIETY, Chemical Communications, no. 16, 15th August 1986, pages 1280-1281, Royal Society of Chemistry; J.E. BALDWIN et al.: "Penicillin Biosynthesis: Structure-reactivity profile of allenic substrates for isopenicillin N synthetase"**

(73) Proprietor: **THE CHANCELLOR, MASTERS AND SCHOLARS OF THE UNIVERSITY OF OXFORD**
**Wellington Square**
**Oxford OX1 2JD(GB)**

(72) Inventor: **Baldwin,Jack Edward**
**10 Rolfe Place**
**Headington Oxford(GB)**

(74) Representative: **Hudson, Christopher Mark**
**Erl Wood Manor**
**Windlesham Surrey GU20 6PH(GB)**

EP 0 268 343 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing 3-substituted cephalosporins. In particular, it relates to an enzymatic process for converting a 2α-substituted penam to a 3-substituted-3-cephem-4-carboxylic acid comprising the use of the enzyme expandase.

The enzyme expandase, also known as deacetoxycephalosporin C synthetase, is involved in the biosynthesis of cephalosporin C by Cephalosporium acremonium as well as by other organisms. Expandase converts penicillin N to deacetoxycephalosporin C as shown in the biosynthetic pathway outlined below.

δ-(L-α-aminoadipoyl)-L-cysteinyl-D-valine

↓ isopenicillin N synthetase

isopenicillin N

↓ epimerase

penicillin N

↓ expandase

deacetoxycephalosporin C

↓ hydroxylase

desacetylcephalosporin C

↓ acetyl transferase

cephalosporin C.

Expandase has been isolated and studied by Scheidegger, A., et al., J. Antibiotics, 37, 522-531 (1984) and by Kupka, J., et al., FEMS Microbiology Letters, 16 (1983) pp. 1-6.

The present invention is based on the discovery that the m-carboxyphenylacetyl group can be substituted for the α-aminoadipoyl group of penicillin N to provide a penam substrate which is efficiently converted by expandase to the cephalosporin. Thus, 6β-(3-carboxyphenylacetylamino)-2,2-dimethylpenam-3-carboxylic acid is efficiently converted to 7β-(3-carboxyphenylacetylamino)-3-methyl-3-cephem-4-carboxylic acid. In addition, the adipoyl group likewise can be substituted for the α-aminoadipoyl group of penicillin N to provide a penam substrate which is efficiently converted by expandase.

In contrast to the above, it has been found that the phenylacetyl group fails to function as a replacement for the α-aminoadipoyl group of penicillin N. Other acyl groups which when attached to the 6-amino group of 6-APA fail to form penam substrates for expandase are phenoxyacetyl, δ-(L-α-aminoadipoyl), and 5-aminovaleryl.

According to this invention, there is provided a process for preparing a 3-substituted-3-cephem-4-carboxylic acid represented by the formula 1

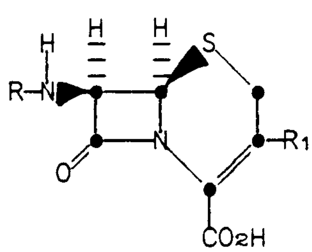

1

wherein R is 3-carboxyphenylacetyl or adipoyl and $R_1$ is $C_1$-$C_3$ alkyl, $C_1$-$C_3$ alkoxy, $C_{2-4}$ alkenyl or allenyl; which comprises contacting in an aqueous medium at a temperature between about 20 °C. and about

40°C. and at a pH of between about 6 and about 9, a 2β-methyl-2α-substituted penam-3-carboxylic acid represented by the formula 2,

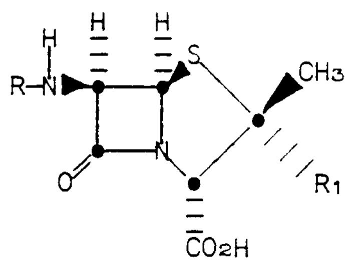

2

wherein R and $R_1$ have the same meanings as defined above, with deacetoxycephalosporin C synthetase in the presence of ferrous ion, ascorbate, and α-ketoglutarate.

The process is carried out in the presence of oxygen. On a small laboratory scale, sufficient oxygen is supplied by using an open reaction vessel. On a large scale, particularly when a large excess of the enzyme is used, oxygen may be supplied by passing air or oxygen into the incubation mixture. The incubation mixture is well agitated by stirring or shaking during the process.

The expandase enzyme employed in the process is preferably purified; however, semipurified enzyme such as is obtained from partially purified cell-free extracts of the enzyme can be used. The expandase is available from numerous microorganisms, e.g. Cephalosporium acremonium, Streptomyces clavuligerus and Streptomyces lipmanii. Strain(s) of C. acremonium (such as strains ATCC 48272 and ATCC 36225) that produce high titers of cephalosporin C are suitable sources of the enzyme. Cell-free extracts of the enzyme are obtained by known methods, e.g. as described by A. Scheidegger et al., supra. Preferably, the enzyme is used in substantially pure form, e.g. as obtained by chromatographic separation of the enzyme from partially purified cell-free extracts.

The expandase is preferably used in large excess of the amount required for conversion of the penicillin to the cephalosporin.

As is known with the conversion of penicillin N by expandase, the use of cofactors, ferrous ion and ascorbate and cosubstrate α-ketoglutarate are required for the best expression of enzyme activity. Accordingly, these cofactors are employed in the present process. The minimum amount of ferrous ion required to activate the enzyme is used. The concentration of ferrous ion is generally between about 25 μM to about 0.2 mM. Generally, the greater the purity of the enzyme the lower the amount of ferrous ion required for maximum enzyme efficiency. Sources of ferrous ion are the salts such as ferrous chloride, ferrous sulfate, or ferrous carbonate. Ferrous sulfate is a preferred salt for use in the process.

The cofactor ascorbate is preferably L-ascorbic acid or sodium or potassium L-ascorbate and is generally used at a concentration about equal to the ferrous ion concentration, although higher concentrations may be employed.

The cosubstrate α-ketoglutarate is preferably in the form of the monosodium or monopotassium salt and is preferably present at a concentration between about 0.5 mM and about 2 mM, preferably about 1 mM.

The substrate penicillin represented by the above formula 2 is generally used in the process at a concentration between about 0.1 mM to about 5 mM.

The process is carried out at a pH of between about 6 and about 9 and preferably at about 7.5 to about 8. The pH is maintained with buffer such as ammonium carbonate, Tris buffer, or MOPS buffer. A preferred buffer is Tris-HCl (pH 7.5).

The process is preferably carried out at a temperature between about 25°C. and 35°C. An especially preferred temperature is about 30°C.

A reducing agent such as dithiothreitol (DTT), dithioerythritol or β-mercaptoethanol is optionally used in the process. Such a reducing agent appears to preserve the activity of the enzyme by inhibiting oxidation of the ferrous ion as well as by inhibiting the oxidation of sulfhydryl groups present in the enzyme.

Examples of 2α-substituted penam substrates represented by the formula 2 are shown below in Table 1.

TABLE 1

2α-Substituted-Penams

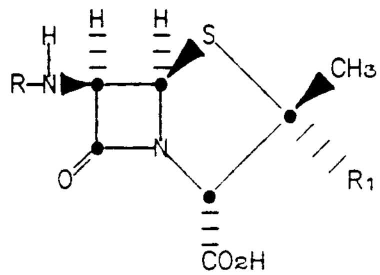

| R | $R_1$ |
| --- | --- |
| CPA[1] | $-CH_3$ |
| " | $-C_2H_5$ |
| " | $-C_3H_7$-n |
| adipoyl[2] | $-CH_3$ |
| adipoyl | $C_2H_5$ |
| " | $-OCH_3$ |
| " | $-OC_2H_5$ |
| " | vinyl |
| " | allenyl |
| CPA | " |
| " | vinyl |
| " | $-OCH_3$ |
| " | $-OC_2H_5$ |
| " | $-O-C_3H_7$-n |
| " | $-O-C_3H_7$-i |

1/CPA = 3-carboxyphenylacetyl

2/adipoyl = $HO_2C(CH_2)_4$-CO-

Preferred substrates in the process are represented by the formula 2 wherein R is the 3-carboxyphenylacetyl group. A further preferred group of substrates is represented when R is the 3-carboxyphenylacetyl group and $R_1$ is methyl, methoxy or vinyl.

The penam substrates can be prepared by the acylation of the 2α-substituted-6β-aminopenam-3-carboxylic acid with a 3-carboxy-protected-phenylacetic acid or a mono-carboxy-protected adipic acid as shown in the following general scheme.

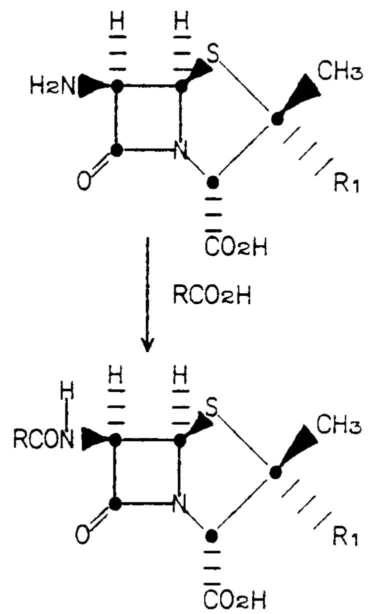


The N-acylation can be carried out by the conventional coupling methods commonly used to prepare penicillins. For example, the carboxy-protected acid, $RCO_2H$ is converted to an active derivative of the free carboxy group such as an acid halide, acid azide, or active ester and the active derivative used to acylate. Preferably, the 3-carboxy group of the penicillin nucleus is protected during the acylation. After the acylation is completed the carboxy groups are deprotected to provide the desired substrate. For example, 3-(4-nitrobenzyloxycarbonyl)phenylacetic acid is converted to the acid chloride with oxalyl chloride and the acid chloride used to acylate 6-APA in an aqueous medium containing a water miscible organic solvent and a water soluble acid binding agent such as an alkali metal carbonate or bicarbonate. Likewise, the mono-(protected carboxy) adipic acid, e.g. adipic acid mono-4-nitrobenzyl ester, is converted to a more facile acylating agent such as the acid halide which is in turn used to acylate the 6β-aminopenam nucleus.

The 2α-vinyl-6β-amino nucleus can be obtained as described in my pending application U.S. Serial No. 856,997, filed April 29, 1986. As described therein, δ-(L-α-aminoadipoyl)-L-cysteinyl-D-γ,δ-didehydroisoleucine represented by the formula

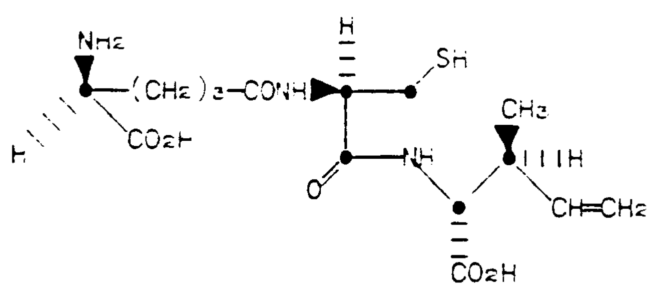

is incubated with isopenicillin N synthetase (IPNS) enzyme at a temperature between about 20 °C. and about 40 °C. at pH 6 to 9 in the presence of ferrous ion and ascorbic acid to form 6β-(L-α-aminoadipoyl)-2α-vinyl-2β-methylpenam-3-carboxylic acid. The carboxy groups of the product penam are protected by esterifcation and the diester is deacylated to provide the 6β-amino-2α-vinylpenam ester. The carboxy-protecting ester group can be a carboxy-protecting ester group commonly used to form penicillin esters such as t-butyl, haloalkyl, e.g. trichloroethyl, benzyl, substituted benzyl, e.g. p-methoxybenzyl and p-nitrobenzyl. The deacylation of the L-α-aminoadipoyl side chain is accomplished by the known procedures, e.g. as described by U.S. Patent No. 3,499,909, or as described by R. B. Morin et al., J. Amer. Chem. Soc., 84, 3400. Other alkenyl derivatives can be similarly prepared.

The 6β-amino-2α-allenylpenam nucleus represented by the formula

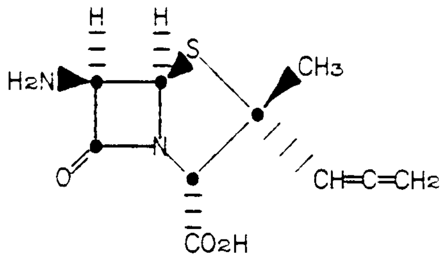

can be obtained as described in my pending application U.S. Serial No. 891,434 filed July 28, 1986. According to the process described therein, a tripeptide substrate represented by the formula

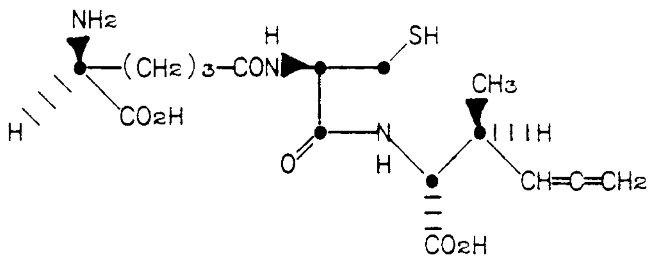

is mixed in an aqueous medium at a pH between about 6 and about 9 with isopenicillin N synthetase in the presence of ferrous ion, L-ascorbate and dithiotreitol to provide the 2α-allenylpenam represented by the formula

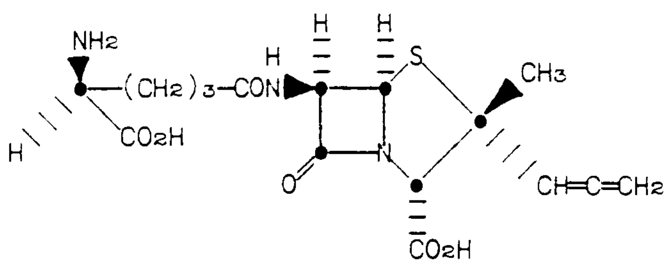

The product is N-deacylated by the method described above for the preparation of 6β-amino-2α-vinylpenam-3-carboxylic acid to provide the 6β-amino-2α-allenylpenam-3-carboxylic acid nucleus.

The 2α-($C_1$-$C_3$ alkoxy)penam nucleus is similarly obtained by the method described in my published European Patent Application No. 0174129. As described therein, a tripeptide substrate represented by the formula

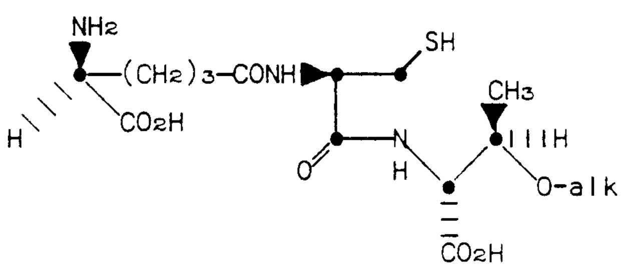

is mixed in an aqueous medium at a pH between about 6 and about 9 with isopenicillin N synthetase in the presence of ferrous ion, L-ascorbic acid, dithiothreitol, beef liver catalase and ammonium bicarbonate buffer to provide the penam represented by the formula

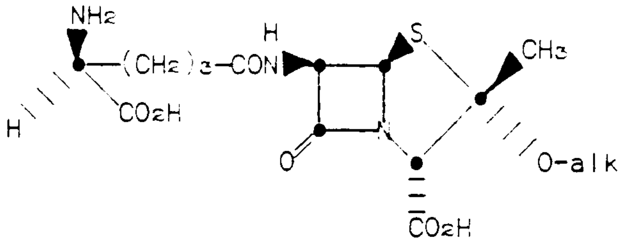

wherein alk is $C_1$-$C_3$ alkyl.

The N-deacylation of the 2α-alkoxypenam is best carried out by the nitrosyl chloride method of R. B. Morin supra and by substituting acetic acid for the formic acid generally used in the Morin method.

Alternatively, the penam substrates (formula 2) wherein R is 3-carboxyphenylacetyl are obtained by the process described by my copending application U.S. Serial No. 903,548, filed on September 4, 1986 (Attorney Docket No. X-7084). According to the process, an N-(3-carboxyphenylacetyl)-L-cysteinyl-D-valine (or modified D-valine) dipeptide represented by the formula

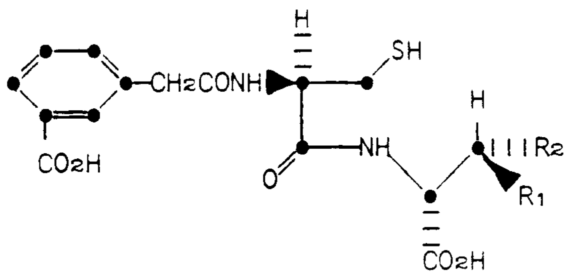

is treated with the enzyme isopenicillin N synthetase (IPNS) in an aqueous medium at a pH between about 6 and about 9 in the presence of oxygen, ferrous ion and L-ascorbic acid; to form the penicillin represented by the formula

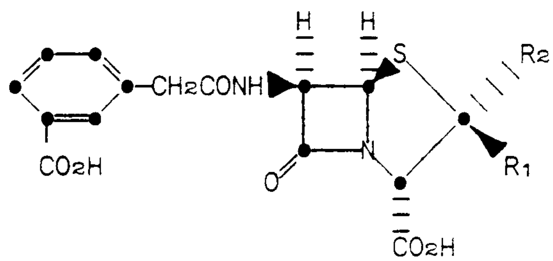

wherein among other groups, $R_1$ or $R_2$ can be methyl and the other $C_1$-$C_4$ alkoxy, vinyl or allenyl.

In an example of the process, an aqueous mixture of N-(3-carboxyphenylacetyl)-L-cysteinyl-D-valine containing dithiothreitol, ferrous sulfate, L-ascorbic acid, catalase, and buffered with ammonium bicarbonate is treated with isopenicillin N synthetase in Tris-HCl buffer to provide 6β-(3-carboxyphenylacetylamino)-2,2-dimethylpenam-3-carboxylic acid.

In like manner and with the appropriately substituted dipeptide substrate, there are obtained 6β-(3-carboxyphenylacetylamino)-2α-vinyl-2β-methylpenam3-carboxylic acid, 6β-(3-carboxyphenylacetylamino)-2 α-allenyl-2β-methylpenam-3-carboxylic acid, and 6β-(3-carboxyphenylacetylamino)-2α-methoxy-2β-methylpenam-3-carboxylic acid.

The 3-substituted-3-cephem-4-carboxylic acids provided by the process of this invention (formula 1) are exemplified below in Table 2.

TABLE 2

| 3-Substituted-3-Cephem-4-Carboxylic Acids | |
|---|---|
| R | $R_1$ |
| CPA[1] | $-CH_3$ |
| " | $-OCH_3$ |
| " | $-OC_2H_5$ |
| " | $-C_2H_5$ |
| " | vinyl |
| " | allenyl |
| " | $-OC_3H_7$-n |
| " | $-OC_3H_7$-i |
| adipoyl | $-CH_3$ |
| " | $-OCH_3$ |
| " | vinyl |
| " | allenyl |
| " | $-C_3H_7$-n |

[1]/CPA = 3-carboxyphenylacetyl

The cephalosporin products obtained in the enzymatic process of this invention can be converted to known cephalosporin semi-synthetic antibiotics by known methods. For example, the 3-carboxyphenylacetyl group and the adipoyl group of the product is deacylated to provide the corresponding 3-substituted 7β-amino-3-cephem nucleus. The latter then is reacylated to provide the desired 7β-acylamino cephalosporin. The foregoing is depicted by the following general reaction scheme.

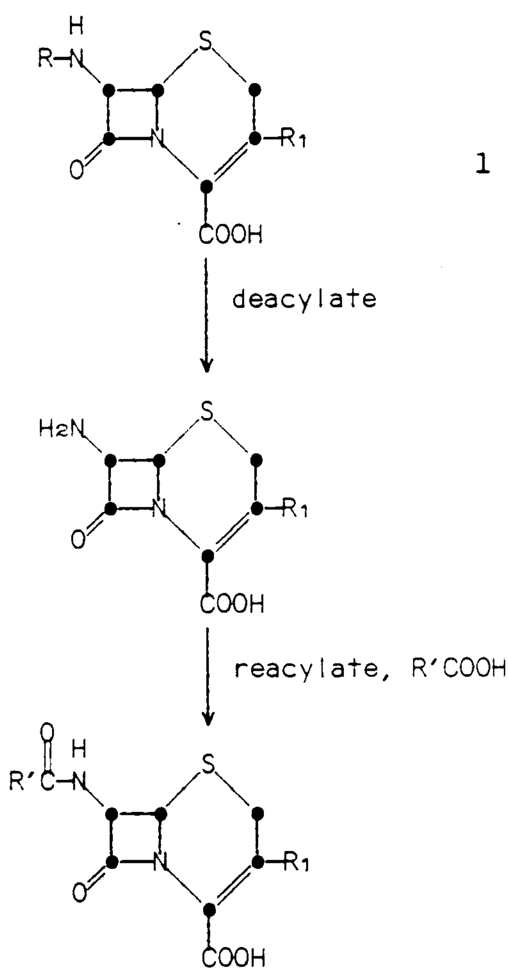


1

The deacylation and reacylation are desirably carried out following protection of the free carboxy groups of 1. The carboxy groups can be protected or blocked with ester groups commonly employed in the cephalosporin art for the temporary protection of the acidic carboxy function. Such well-known ester groups include those described above in connection with the protection of the carboxy function of penams.

The N-deacylation is carried out according to the method described by Fechtig, et al., U.S. Patent No. 3,697,515. The deacylation is accomplished by reaction of the cephalosporin (formula 1) as the diester with an imino halide forming reagent such as a phosphorus halide, e.g. phosphorous trichloride or phosphorus pentachloride. The imino halide is treated with a lower alcohol, e.g. methanol or isobutanol with formation of the corresponding imino ether. The latter is hydrolyzed or decomposes to form the 7β-amino nucleus compound. In an example of the process, p-nitrobenzyl 7β-[3-(p-nitrobenzyloxycarbonyl)-phenylacetylamino]-3-methyl-3-cephem-4-carboxylate is treated in methylene chloride with $PCl_5$ in the presence of triethylamine at a temperature of about 5°C. to form the imino chloride. The mixture is treated in the cold with methyl alcohol or isobutanol to form the imino ether. The reaction mixture then is allowed to warm to room temperature and water is added to form the 7β-amino nucleus ester. The nucleus can be isolated as the hydrochloride salt by conventional methods.

The p-nitrobenzyl 7β-amino-3-methyl-3-cephem-4-carboxylate is reacylated by conventional procedures to provide the desired 7β-acylamino semi-synthetic cephalosporin antibiotic. For example, the nucleus is acylated with the mixed anhydride of an enamine-protected D-phenylglycine formed with ethyl chloroformate and ethyl acetoacetate and represented by the formula

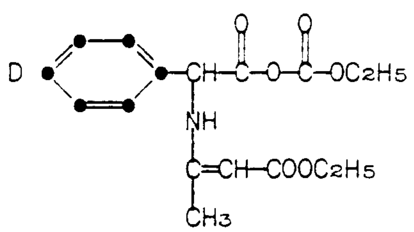

to form the amino-protected derivative of cephalexin p-nitrobenzyl ester represented by the formula

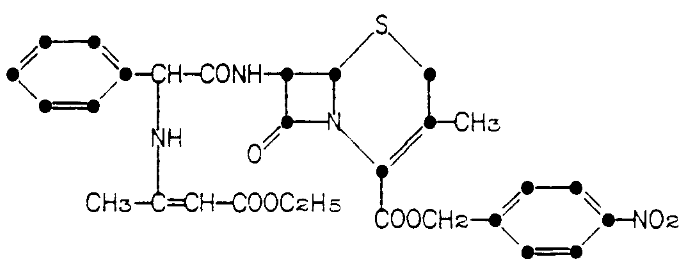

The derivative is treated with zinc and acid (HCl) to remove both the ester group and the enamine-protecting group to provide the well-known orally effective antibiotic cephalexin. Other known cephalosporin antibiotics likewise are obtained by known; acylation methods. Examples of the 7β-amino-3-cephem nuclei thus obtainable with the products of the enzymatic process include 7-amino-3-methyl-3-cephem-4-carboxylic acid (7-ADCA), 7-amino-3-ethyl-3-cephem-4-carboxylic acid, 7-amino-3-methoxy-3-cephem-4-carboxylic acid, 7-amino-3-vinyl-3-cephem-4-carboxylic acid, and 7-amino-3-allenyl-3-cephem-4-carboxylic acid.

The 7-amino-3-cephem nucleus compounds are known. For example, 7-ADCA is described by Stedman, J. Med. Chem., 7, 117 (1964); the 3-methoxy nucleus by Chauvette, U.S. Patent No. 3,917,587; and the 3-vinyl nucleus by U.S. Patent No. 3,994,884.

The 3-allenyl-3-cephem nucleus compounds provided via the process of this invention as described above are a further aspect of this invention. These nuclei are represented by the formula

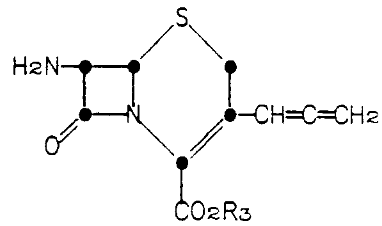

wherein $R_3$ is hydrogen or a carboxy-protecting group. Preferred carboxy-protecting groups are represented when $R_3$ is t-butyl, benzyl, 4-methoxybenzyl, 4-nitrobenzyl, diphenylmethyl, or a tri-($C_1$-$C_4$ alkyl)silyl group such as trimethylsilyl or dimethyl-t-butylsilyl.

The following Preparations and Examples are provided to further describe the invention.

I Preparation of 2α-substituted-penams via isopenicillin N synthetase.

2α-Vinyl-2β-Penam

Preparation 1

Preparation of δ-(L-α-aminoadipoyl)-L-cysteinyl-D-γ,δ-didehydroisoleucine

A. Benzyl 2-(t-butyloxycarbonylamino)-3-methylpent-4-enoate

A mixture of 4.4 mmol of 2-(t-butyloxycarbonylamino)-3-methylpent-4-eneoic acid (2R,3S; 3S,2R) prepared by the method of P. A. Bartlett et al., J. Org. Chem., 1982, 47, 3933, in 10 ml of dry DMF containing 4.4 mmol of benzyl bromide, 4.4 mmol of sodium bicarbonate and 10 mg. of sodium iodide was stirred for 15 minutes at 20 °C. The mixture was dissolved in ethyl acetate, the solution washed three times with water, was dried over sodium sulfate, filtered and evaporated. The ester product was purified by flash chromatography on silica gel. The ester was obtained in an 80% yield as an oil.

NMR (300 MHz, $CHCl_3$): δ1.01 (3H, d, J=6Hz, 3-$CH_3$), 1.44 (9H, s, t-butyl), 2.58-2.71 (1H, m, 3-H), 4.32-4.40 (1H, m, 2-H), 5.01-5.27 (5H, m, 5-H, NH, $CH_2C_6H_5$), 5.05-5.37 (1H, m, 4-H), 7.33-7.41 (5H, m, $C_6H_5$-H).

IR ($CHCl_3$): $\lambda_{max}$ 3005 m, 1730 s ($CO_2$), 1710 s ($CO_2$), 1600 w, 1502 m, 1205 s, 1160 m $cm^{-1}$.

m/e ($NH_3$, Desorption Chemical Ionization): 320 ($MH^+$, 100%)

B. Benzyl 2-amino-3-methylpent-4-eneoate formic acid salt

The benzyl ester (step A, 0.40 mmol), was dissolved in 1 ml. of formic acid and the solution stirred for 2 hours at 20 °C. The solution was evaporated to yield the crude formic acid salt which was used in the next step without further purification.

C. The benzyl ester formic acid salt (step B) was coupled with the protected α-aminoadipoyl-L-cysteinyl dipeptide represented by the formula

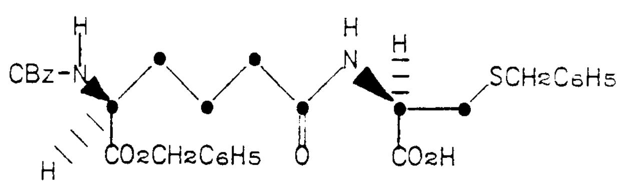

using the standard coupling conditions with EEDQ as described by J. E. Baldwin et al., J. Chem. Soc. Perkin 1, 1981, 2253. The S-benzyl, CBz-protected title tripeptide dibenzyl ester was obtained in 50% yield as a 1:1 mixture with the corresponding diastereoisomer, the allo-isoleucinyl tripeptide.

The protected title tripeptide was separated from the diastereoisomeric mixture by plate layer chromatography on silica gel using 4:6, (v:v) ethyl acetate:hexane, as eluant. The protected title tripeptide was crystallized to homogeneity from ethyl acetate:hexane and melted at 116 °C. to 118 °C.

IR ($CHCl_3$): $\lambda_{max}$ 1730 s ($CO_2$), 1700 s ($CO_2$), 1508 m, 1378 m, 1205 w.

NMR (300 MHz, $CHCl_3$): δ1.00 (3H, d, J=6 Hz, $CHCH_3$), 1.61-1.92 (4H, m, $CH_2$-$CH_2CH_2CO$), 1.07-1.25 (3H, m, $CH_2$-CO, $CHCH_3$), 2.58-2.88 (2H, m, $CH_2S$), 3.75 (2H, s, $SCH_2C_6H_5$), 4.36-4.48 (2H, m, 2 × NHCHCO), 4.57-4.63 (1H, m, NHCHCO), 4.99-5.21 (8H, m, 3 × $OCH_2C_6H_5$, C=$CH_2$), 5.56-5.75 (2H, m,

CH = $CH_2$, NH), 6.35 (1H, d, J = 8 Hz, NH), 6.91 (1H, d, J = 8.5 Hz, NH), 7.31-7.37 (20H, m, aryl H).

m/e (field desorption) 779 ($M^+$).

The structure of the separated title compound was further shown by its reduction in benzene for one hour with hydrogen at 1 atmosphere using $[(C_6H_5)_3P]_3RhCl$, to the corresponding allo-isoleucinyl tripeptide described by J. E. Baldwin et al., J. Chem. Soc., Chem. Commun., 1984, 1167, and references cited therein.

D. Deprotection of title tripeptide

The N-CBz S-benzyl protected tripeptide dibenzyl ester (step C) was deprotected in sodium/liquid ammonia under the conditions described by J. E. Baldwin et al., J. Chem. Soc., Perkin 1, 1981, 2253, to provide the title tripeptide.

The deprotected tripeptide was then oxidized to the disulfide in dilute ammonium hydroxide, pH 8, by passing oxygen gas through the solution for 2 hours. The disulfide was purified by preparative electrophoresis (pH 3.5, 4KV, 80 minutes) and by extracting the ninhydrin-active band that migrated 5-10 cm towards the anode. The purified disulfide was obtained in 77% yield.

NMR (300 MHz, $D_2O$, HOD = 4.63 p.p.m.): δ0.87 (3H, d, J = 5.5 Hz, $CHCH_3$), 1.51-1.74 (4H, m, $CH_2CH_2CH_2CO$), 2.08-2.26 (2H, m, $CH_2CO$), 2.55-2.57 (1H, m, $CHCH_3$), 2.77-3.03 (2H, m, $CH_2S$), 3.47-3.58 (1H, m, NHCHCO), 4.14 (1H, d, J = 5.5 Hz, NHCHCO), 4.91-4.96 (2H, m, C = $CH_2$), 5.60-5.67 (1H, m, CH = $CH_2$).

m/e (positive argon F.A.B., in presence of dithiothreitol) 376 ($MH^+$, 12%).

## Preparation 2

6β-(L-α-Aminoadipoyl)-2β-methyl-2α-vinylpenam-3-carboxylic acid

An aqueous solution of the δ-(L-α-aminoadipoyl)-L-cysteinyl-D-γ,δ-didehydroisoleucine disulfide prepared as described by Preparation 1 (28 mM, 0.100 ml) was mixed with aqueous solutions of dithiothreitol (100 mM, 0.100 ml), L-ascorbic acid (50 mM, 0.100 ml), ferrous sulfate (5 mM, 0.100 ml), bovine liver catalase (10,000 units/ml, 0.050 ml) and ammonium bicarbonate (50 mM, 3.5 ml). The pH of the mixed solutions was monitored and, when necessary, adjusted to 8 by adding dilute sodium hydroxide (100 mM). The mixed solutions were shaken at 27° C. for 5 minutes and an isopenicillin N synthetase preparation isolated from Cephalosporium acremonium (5 I.U./ml, 1 ml) in a 50 mM solution of ammonium carbonate was added. The incubation mixture was shaken in two 10 ml vials at 27° C. for 45 minutes and then the reaction was terminated by precipitating the protein with 7 ml of acetone. The precipitate was separated by centrifugation. The supernatant was evaporated in vacuo to remove the acetone and the residue was freeze-dried to yield the crude 2α-vinylpenam represented by the formula

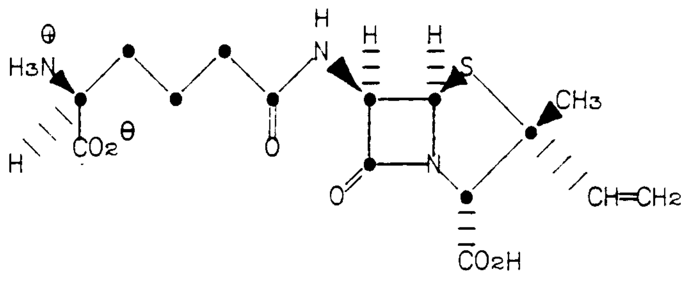

The crude product was purified via reverse phase octadecylsilane HPLC (250 × 4.6 mm column); mobile phase: 10 mmolar aqueous ammonium bicarbonate; flow 1 ml $min.^{-1}$; retention time = 7-8 minutes.

NMR (500 MHz, $D_2O$, 3-trimethylsilylpropionate [2,2,3,3-$^2H_4$] TSP = 0.00 p.p.m.): δ1.53-1.77 (4H, m, $CH_2CH_2CH_2CO$), 1.59 (3H, s, 3β-$CH_3$), 2.25-2.38 (2H, m, $CH_2CO$), 3.56-3.59 (1H, m, $CH(CH_2)_3$), 4.18 (1H, s, 2-H), 5.03 (1H, d, J = 10.5 Hz, CH = $CH_2$), 5.22 (1H, d, J = 17 Hz, CH = $CH_2$), 5.35, 5.45 (2H, ABq, J = 4Hz, 5, 6-H), 5.91 (1H, dd, J = 10.5, 17 Hz, CH = $CH_2$).

The assignment of the chemical shift for the 2β-methyl group follows from Nuclear Overhauser experiments. Thus, irradiation of the 2β-$CH_3$ group, at δH 1.59, gave enhancement (12%) of 2-H but not of 5-H, whereas irradiation of the CH = $CH_2$ proton, at δH 5.91, gave enhancement of 5-H (3%).

m/e (positive argon fast atom bombardment) 372 ($MH^+$).

## Preparation 3

6β-Amino-2α-vinyl-2β-methylpenam-3-carboxylic acid

6β-(L-α-aminoadipoyl)-2β-methyl-2α-vinylpenam-3-carboxylic acid is converted to the N-phthaloyl derivative and the sodium salt of the derivative is suspended in dry methylene chloride. The suspension is treated with trimethylchlorosilane in the presence of pyridine to form the trimethylsilyl ester. The silyl ester derivative is reacted at about -5°C. with phosphorous pentachloride to form the corresponding imino chloride. The reaction mixture is cooled to about -20°C. and is treated with methyl alcohol to form the corresponding imino ether. The mixture is allowed to warm to about 0°C. and water is added to form the title nucleus compound. The mixture is poured into a cold mixture of water and methyl alcohol and the aqueous layer is separated. After adjustment of the pH to about 4, the solution is allowed to stand at about 5°C. to precipitate the title nucleus compound.

2α-Allenyl-2β-Penams

Preparation 4

The 6β-amino-2α-allenyl-2β-methylpenam-3-carboxylic acid is obtained as follows. 2R,3R,-2-(t-Butyloxycarbonylamino)-2-iodobutanoic acid diphenylmethyl ester is mixed in dry degassed benzene with excess triphenylpropargyl tin and azobis(isobutyronitrile) (AIBN) and the solution is heated at the reflux temperature under nitrogen for about 18 hours. The mixture is cooled, evaporated to dryness, and the residue is taken up in acetonitrile and washed with hexane. Following evaporation the residue of product is chromatographed over silica gel to provide diphenylmethyl 2-(t-butyloxycarbonylamino)-3-methyl-4,5-hexadienoate.

The amino-protecting t-BOC group is removed from the product with anhydrous p-toluenesulfonic acid in cold ethanol and the free amino ester is coupled with amino-protected and S-protected δ-(L-α-aminoadipoylamino)-L-cysteine diester represented by the formula

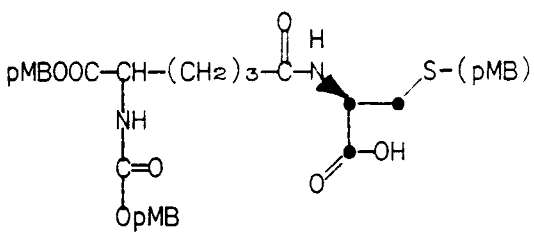

wherein pMB is p-methoxybenzyl. The coupling is carried out under nitrogen in dry dichloromethane at room temperature with 1-ethoxycarbonyl-2-ethoxy-1,2-dihydroquinoline (EEDQ) in the presence of triethylamine.

The protected tripeptide product obtained is deprotected by heating with trifluoroacetic acid and anisole at the reflux temperature for about 30 minutes to provide the tripeptide represented by the formula

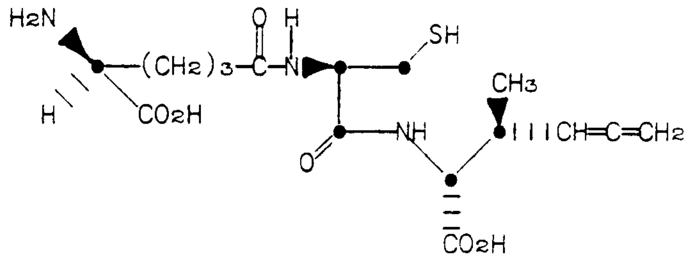

The tripeptide is incubated with excess isopenicillin N synthetase at about 27°C. in an aqueous medium (pH 7.7-8.0) containing dithiothreitol, L-ascorbic acid, ferrous sulfate, and beef liver catalase. After about one hour the reaction is terminated by the addition of acetone. The precipitated protein is separated by centrifugation and the supernatant containing the product is evaporated to remove acetone and the remaining aqueous solution is freeze-dried to provide a lyophile of the product represented by the formula

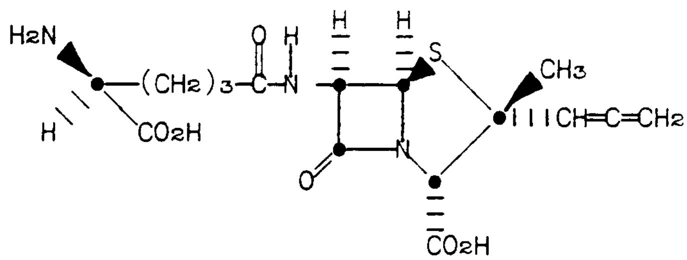

The 2α-allenylpenam is deacylated by following the procedures of Preparation 3.

2α-alkoxypenams

Preparation 5

δ-(L-α-aminoadipoyl)-L-cysteinyl-(2R,3R)-2-amino-3-methoxybutanoic acid

(1) 2R,3R- and 2S,3S-2-Bromo-3-methoxybutanoic acid

Crotonic acid (8.6 g, 100 mmol) is dissolved in methanol (50 ml) and N-bromacetamide (13.8 g, 10 mmol) is added in portions over 30 minutes. The solution is stirred at 25° C. for 15 hours and the solvent is then evaporated and the residue partitioned between diethyl ether and water. The ether layer is dried ($Na_2SO_4$), filtered, evaporated and the residue distilled to yield the title compounds as a colorless oil (14.3 g, 72%), b.p. 88-89°/0.5 mm.

(2) 2R,3R- and 2S,3S-2-Amino-3-methoxybutanoic acid

2R,3R- and 2S,3S-2-Bromo-3-methoxybutanoic acid (14 g, 71 mmol) are dissolved in ammonia (s.g. 0.88, 250 ml) and the mixture heated in an autoclave at 95° C. for 8 hours. The mixture is cooled to 25° C., evaporated, and the residue suspended in acetone. The resultant colorless solid is filtered off and washed with acetone to give the title compounds as a colorless solid containing residual ammonium bromide, (12.1 g) m.p. 180-184° C. (dec.).

(3) 2R,3R- and 2S,3S-N-Benzyloxycarbonyl-2-amino-3-methoxybutanoic acid, benzyl ester

2R,3R- and 2S,3S-2-Amino-3-methoxybutanoic acid (3.1 g) are dissolved in a mixture of 1M sodium hydroxide (27 ml), water (20 ml), and dioxan (40 ml). Benzyl chloroformate (3.8 ml, 22 mmol) in dioxan (20 ml) and 1M sodium hydroxide (27 ml) are added to the solution individually, each at the same rate of addition, over 25 minutes. The mixture is stirred for 1 hour, extracted into ethyl acetate (3 × 200 ml), acidified to pH 1 with 2N hydrochloric acid and then reextracted into ethyl acetate (3 × 200 ml). The combined organic extracts are dried, filtered, and evaporated to give an oil. This oil is dissolved in dry dimethyl formamide (20 ml) and to the solution are added sodium hydrogen carbonate (3.14 g, 41 mmol), benzyl bromide (3.8 ml, 33 mmol), anhydrous sodium sulfate (200 mg) and sodium iodide (10 mg), the whole being stirred at 25° C. for 24 hours. The mixture is then extracted into dichloromethane (200 ml), washed with water (4 × 300 ml), dried (sodium sulfate), filtered and evaporated. Purification by chromatography on silica gel using ethyl acetate and petroleum ether as consecutive eluants gives the title compounds as a colorless oil (4.2 g, 65%) which solidifies on standing, m.p. 44° C.

(4) 2R,3R- and 2S,3S-2-Amino-3-methoxybutanoic acid, benzyl ester

2R,3R- and 2S,3S-N-Benzyloxycarbonyl-2-amino-3-methoxybutanoic acid, benzyl ester (500 mg, 1.4 mmol) are dissolved in dry dichloromethane (3 ml) and hydrobromic acid (45% in acetic acid, 2 ml) is added. The mixture is stirred under argon for 20 minutes and is then evaporated. The residue is dissolved in dichloromethane (3 × 3 ml) and reevaporated (3×) to give a further residue which is dissolved in xylene (2 × 3 ml) and reevaporated (2×). Petroleum ether (5 ml) is then added and the product is triturated for 5 minutes before discarding the mother liquor and dissolving the solid residue in dichloromethane (5 ml). Triethylamine (1 ml) is added to the dichloromethane solution which is then stirred for 2 minutes and

evaporated. The residue is dissolved in dichloromethane (3 × 5 ml) and reevaporated (3×) to give a further residue which is triturated with diethyl ether (5 ml). The resulting solid is filtered off, reextracted with diethyl ether (2 × 5 ml) and the ethereal layers combined and evaporated to yield the title compounds as a colorless oil (290 mg, 93%).

(5) (N-Benzoyloxycarbonyl-α-benzyl-δ-L-aminoadipoyl)-S-benzyl-L-cysteinyl-(2R,3R-2-amino-3-methoxybutanoic acid), benzyl ester

2R,3R- and 2S,3S-2-Amino-3-methoxybutanoic acid, benzyl ester (177 mg, 0.79 mmol) are dissolved in dry dichloromethane (10 ml) and 2-ethoxy-N-ethoxycarbonyl-1,2-dihydroquinoline (195 mg, 0.79 mmol) and N-benzyloxycarbonyl-α-benzyl-δ-(L-α-aminoadipoyl)-S-benzyl-L-cysteine (456 mg, 0.79 mmol), (prepared as described by Baldwin et al., Journal of the Chemical Society, Perkin I, 1981, 2253), and sodium sulfate (100 mg) are added and the mixture stirred for 24 hours. The solution is evaporated, the residue is dissolved in ethyl acetate (150 ml), and the ethyl acetate solution washed in turn with 2M hydrochloric acid (50 ml), saturated sodium hydrogen carbonate solution (50 ml) and brine (50 ml), then dried, filtered and evaporated. Purification by chromatography on silica using ethyl acetate and hexane as consecutive eluants gives the title compound as a colorless solid (180 mg, 29%), m.p. 116-118°; $[\alpha]_D^{20}$ -11.7° (c 1, $CHCl_3$) (the 2R,3R isomer is less polar than the 2S,3S, isomer).

(6) δ-(L-α-Aminoadipoyl)-L-cysteinyl-(2R,3R-2-amino-3-methoxybutanoic acid)

N-Benzyloxycarbonyl-α-benzyl-δ-(L-α-aminoadipoyl)-S-benzyl-L cysteinyl-(2R,3R-2-amino-3-methoxybutanoic acid), benzyl ester electrophoresis (pH 3.5, 3 Kv, 2 hours) gives the title compound in its disulfide form (10 mg, 26%) as a colorless solid, $^1$H n.m.r. ($D_2O$, referred to external TMS) δ 1.5-1.8 (4H,m), 2.25 (2H, t, J = 7.0 Hz) 2.73 (2H, m), 3.19 (3H, s), 3.65 (2H, ABX, $J_{AB}$ = 10,5 $J_{AX}$ = 3.9, $J_{BX}$ = 5.5 Hz), 3.84 (1H, t, J = 6.2 Hz), 4.40 (1H, t, J = 5.8 Hz), 4.47 (1H, m).

Preparation 6

6-δ-(L-α-Aminoadipamido)-2α-methoxy-2β-methylpenam-3-carboxylic acid

δ-(L-α-Aminoadipoyl)-L-cysteinyl-( 2R,3R,-2-amino-3-methoxybutanoic acid) (210 μg) is incubated with purified isopenicillin N synthetase (1.1 units), in the presence of the cofactors dithiothreitol (2.11 mM), ascorbic acid (1.06 mM), ferrous sulfate (0.11 mM) and catalase (bovine liver, 1800 sigma units) in Tris-HCl buffer (50 mM, p 7.5; tris stands for 2-amino-2-hydroxymethylpropane 1,3-diol) to a total volume of 1 ml, the incubation being carried out at 27°C. in a shaker (250 r.p.m.) with exposure exposed to the air for 30 minutes. The protein is precipitated from the mixture by the addition of acetone to a concentration of 70% by volume of acetone, then separated by centrifugation and the supernatant freeze-dried. The freeze-dried material is redissolved in water (500 μl) and the product purified by HPLC (Waters:Z Module Radial Compression Separator System with Radial-Pak $C_{18}$10 cartridge) using 90% 50 mM $KH_2PO_4$/10% methanol by volume as the eluting solvent and detecting the antibiotic by its absorption at 220 nm. The product which is thus obtained is freeze-dried immediately to provide the title compound.

Preparation 7

6β-Amino-2α-methoxy-2β-methylpenam-3-carboxylic acid

To a solution of the 2α-methoxy-2β-methylpenam obtained in Preparation 6 (1.15 mg, 3.1 μmole) in acetonitrile (0.3 ml) under nitrogen at 0°C. was added nitrosyl chloride in acetic acid (0.61M, 0.025 ml). After 5 minutes at 0°C. the solution was evaporated to dryness at room temperature. Methanol (0.4 ml) was added, the solution was left standing for 5 minutes and the methanol removed in vacuo. Hydrochloric acid/potassium chloride buffer (0.04M HCl, 0.16M KCl, pH 2, 0.4 ml) was added and the solution was left standing for 3 minutes at room temperature. The solution was neutralized with sodium bicarbonate (70 mM, 0.3 ml) and freeze-dried. Purification by HPLC (reverse phase octadecylsilane column, using 5 mM $NaH_2PO_4$/$Na_2HPO_4$/KCl pH 7 buffer:methanol (19:1) as eluant) gave the title compound (0.050 mg, 7%).

II Preparation of 6β-(3-carboxyphenylacetylamino)-2α-substituted-2β-methylpenam-3-carboxylic acids via isopenicillin N synthetase.

Preparation 8

N-(3-Carboxyphenylacetyl)-L-cysteinyl-D-valine

A. iso-Phthalic acid monobenzyl ester

To a suspension of iso-phthalic acid (11.2 g, 0.1 mole) in methyl alcohol (200 ml) and $H_2O$ (10 ml) was added a solution of potassium hydroxide (11.2 g, 0.2 mole) in methyl alcohol (100 ml), and the mixture was stirred overnight at room temperature. The solvent was removed under reduced pressure and DMF (250 ml) and benzyl bromide (13.5 ml, 1.1 g) were added. The mixture was heated for 2 hours at 100°C., poured into aqueous sodium bicarbonate (10 g in 500 ml of water) and extracted with ethyl acetate. The aqueous layer was acidified (concentrated HCl) and extracted with ethyl acetate and the precipitated iso-phthalic acid was removed by filtration. The ethyl acetate layer was washed with brine, dried ($Na_2SO_4$) and evaporated. The residue (monobenzyl ester and iso-phthalic acid) was chromatographed on silica gel to yield 3.73 g (15%) of the monobenzyl ester.

$^1H$ NMR (300 MHz, $CDCl_3$): δ 5.42 (2H, s), 7.30-7.50 (5H, m), 7.59 (1H, t, J = 7.7 Hz), 8.32 (2H, t, J = 4.5 Hz), 8.81 (1H, s).

IR ($CHCl_3$): 2700-3400 (COOH), 1720 (s), 1700 (s) $cm^{-1}$.

B. 2,2,2-Trichloroethyl 3-(benzyloxycarbonyl)phenylacetate

A mixture of iso-phthalic acid monobenzyl ester (2.6 g), dry benzene (8 ml) and pure thionyl chloride (2.6 ml, 3 eq.) was heated for 1 hour at 80°C. The solvent was removed under reduced pressure and then dry benzene (10 ml) was added and the solvent was again removed under reduced pressure in order to remove traces of unreacted thionyl chloride. The acid chloride was dissolved in dry benzene (20 ml) and the solution was poured into an ethereal solution of diazomethane (excess) at 0°C. The solution was stirred for 20 minutes at 0°C. and then overnight at room temperature. The solvent and excess diazomethane were removed under reduced pressure. To the diazoketone was added 12 ml of 2,2,2-trichloroethanol and to the warm mixture (50 ~ 60°C.) was added silver oxide (0.3 g). Nitrogen was evolved and after 2 hours, additional silver oxide (0.3 g) was added and heating was continued for 30 minutes. A further 0.2 g of silver oxide was added and after 30 minutes, the diazoketone was consumed (by TLC). The mixture was diluted with chloroform, treated with charcoal, filtered and concentrated by evaporation. The residue was purified by flash chromatography [benzene-petroleum ether (1:1)]. There were obtained 3.6 g of the 2,2,2-trichloroethyl ester (88% yield). The diazomethane used was obtained with N-nitrosomethylurea.

$^1H$ NMR ($CDCl_3$, 300 MHz): δ 3.83 (2H, s), 4.76 (2H, s), 5.37 (2H, s), 7.30-7.60 (7H, m), 8.04 (2H, m).

IR ($CHCl_3$): 3020 (m), 2960 (w), 1755 (s), 1740 (s), 1610 (w), 1590 (w), 1500 (w), 1450 (m), 1375 (m), 1280 (s) $cm^{-1}$.

MS m/e: 91 (100), 225 [22, M- ($Cl_3CH_2OC(0)$)], 293 [45, M- Ph($H_2O$)], 295 (42), 297 (14), 400 (7.7, $M^+$ $^{35}Cl_3$), 402 (6.9, M + 2), 404 (2.8, M + 4).

C. 3-(Benzyloxycarbonyl)phenylacetic acid

Zinc dust (7.2 g), followed by 1M aqueous potassium dihydrogen phosphate ($KH_2PO_4$) (7.2 ml) were added to a rapidly stirred solution of the 2,2,2-trichloroethyl ester prepared as described above (3.6 g) in tetrahydrofuran (36 ml) at room temperature. After 15 minutes TLC showed no reaction had occurred. A further 7.2 g of zinc were added. After a few minutes the temperature had increased. After 30 minutes the zinc was filtered off and the solvent was removed under reduced pressure. The residue was dissolved in chloroform and 2N HCl (20 ml) was added and the mixture was stirred for 1 hour. The chloroform layer was separated and the aqueous layer was extracted with chloroform. The extract was combined with the chloroform layer, washed with brine, dried over sodium sulfate and evaporated. The residue was purified by flash chromatography (ethyl acetate) to give 869 mg (37% yield) of 3-(benzyloxycarbonyl)phenylacetic acid.

m.p. 95-96° (from ether)

| Elemental analysis calculated for $C_{16}H_{14}O_4$: | | |
|---|---|---|
| Theory: | C, 71.10; | H, 5.22. |
| Found: | C, 71.22; | H, 5.27. |

$^1H$ NMR (300 MHz, $CDCl_3$): δ 3.72 (2H, s), 5.37 (2H, s), 7.30-7.55 (7H, m), 7.98-8.09 (2H, m).
MS m/e: 91 (34), 163 [100, M- Ph($H_2O$)], 252 (6.7, M- $H_2O$), 270 ($M^+$, 10).
IR ($CHCl_3$): 2800 ~ 3400 (COOH), 1715 (C = O) $cm^{-1}$.

D. Acylation of S-(4-methoxybenzyl)-L-cysteinyl-D-valine diphenylmethyl ester

A mixture of 3-(benzyloxycarbonyl)phenylacetic acid (427 mg, 1.58 mmole), EEDQ (390 mg, 1 equiv.) and S-(4-methoxybenzyl)-L-cysteinyl-D-valine diphenylmethyl ester (1 equiv.) in 10 ml of dry methylene chloride was stirred at room temperature overnight. The solvent was evaporated and the residue was dissolved in 80 ml of ethyl acetate. The solution was washed with aqueous sodium bicarbonate, brine, 10% aqueous citric acid, again with brine and was dried over sodium sulfate. The solvent was evaporated and the residue purified by flash chromatography (20% ethyl acetate-petroleum ether). There were obtained 1.005 g of N-[3-(benzyloxycarbonyl)phenylacetyl]-S-(4-methoxybenzyl)-L-cysteinyl-D-valine diphenylmethyl ester, melting at about 136° C. to about 137° C. (diethyl ether).

| Elemental analysis calculated for $C_{45}H_{46}N_2O_7S$: | | | |
|---|---|---|---|
| Theory: | C, 71.22; | H, 6.11; | N, 3.69. |
| Found: | C, 71.12; | H, 6.02; | N, 3.59. |

$^1H$ NMR ($CDCl_3$, 300 MHz): δ 0.73 and 0.85 (each 3H, d, J = 6.9 Hz), 2.23 (1H, m), 2.62 (1H, dd, J = 14.0, 7.5 Hz), 2.82 (1H, dd, J = 14.0, 5.4 Hz), 3.56 (2H, s), 3.69 (2H, s), 3.76 (3H, s), 4.52 (1H, dd, J = 7.5, 5.4 Hz), 4.63 (1H, dd, J = 8.5, 4.4 Hz), 5.35 (2H, s), 6.35 (1H, d, J = 7.5 Hz), 6.70 (1H, d, J = 8.5 Hz), 6.83 and 7.16 (each 2H, d, J = 8.0 Hz), 6.91 (1H, s), 7.28 ~ 7.48 (17H, m), 7.96 (2H, m).
IR ($CHCl_3$): 3400, 3010, 2960, 1720, 1670, 1610, 1515, 1500 $cm^{-1}$.
MS: 758 ($M^+$).

The triprotected N-acylation product (150 mg, 0.2 mmole) was dissolved in 3 ml of methylene chloride and 0.36 ml of anisole was added. The solution was cooled in an ice bath and 2 ml of a 0.5M solution of aluminum chloride in nitromethane was added. The mixture was stirred for 2 hours at 0° C. and then for 3 hours at room temperature. The reaction mixture was diluted with ethyl acetate, washed with 1N hydrochloric acid and extracted with 5% aqueous sodium bicarbonate. The extract was washed with ethyl acetate, acidified with hydrochloric acid and the deprotected peptide extracted with ethyl acetate. The extract was washed with brine, dried and evaporated to provide 70 mg (93% yield) of the title compound melting at about 196° C. to about 197° C. (decomposition) (from acetone).

| Analysis calculated for $C_{17}H_{22}N_2O_6S$: | | | |
|---|---|---|---|
| Theory: | C, 53.39; | H, 5.80; | N, 7.32. |
| Found: | C, 53.55; | H, 5.82; | N, 7.23. |

$^1H$ NMR (300 MHz, $CD_3OD$): δ 0.89 and 0.94 (each 3H, d, J=6.9 Hz), 2.15 (1H, m), 2.81 and 2.90 (each 1H, dd, J=14.0, 6.5 Hz), 3.67 (2H, s), 4.31 (1H, d, J=5.4 Hz), 4.58 (1H, t, J=6.5 Hz), 7.42 (1H, t, J=7.7 Hz), 7.56 (1H, d, J=7.7 Hz), 7.90 (1H, d, J=7.7 Hz), 8.01 (1H, s).
MS (FAB EX. glycerol/oxalic acid) m/e: 383 (M + 1).
IR (KBr): 3300, 2800 ~ 3300 (broad), 2680 (w), 2560 (w), 1730, 1700, 1650, 1605 $cm^{-1}$.

Preparation 9

6β-(3-Carboxyphenylacetylamino)-2,2-dimethylpenam-3-carboxylic acid via IPNS

A mixture of N-(3-carboxyphenylacetyl)-Lcysteinyl-D-valine (1.4 mg, 3.66 × $10^{-3}$ mmole), 100 µl of 100 mmolar dithiothreitol, 100 µl of 5 mmolar ferrous sulfate, 100 µl of 50 mmolar L-ascorbic acid, 50 µl of catalase (1/10 dilution), 1 ml of 50 mmolar ammonium bicarbonate and 3.5 ml of an isopenicillin N-synthetase solution in 50 mmolar ammonium bicarbonate [prepared from 1 ml of IPNS in Tris•HCl buffer (activity 2700 C.U. $ml^{-1}$, specific activity of 109.8 C.U. $ml^{-1}$)] was divided into two equal volumes and each half placed in an open vial. The contents of each vial were incubated at 30° C. (open to air) at 250 rpm. After 30 minutes 50 µl of 100 mmolar dithiothreitol and 50 µl of 5 mmolar ferrous sulfate were added to

each vial and incubation was continued for 30 minutes. The incubated mixtures were combined, 12 ml of acetone added and the mixture centrifuged. The supernatant was decanted and evaporated to dryness and the residue of product was freeze-dried. The product was purified via reverse phase $C_{18}$ analytical column using 3% acetonitrile-90% 10 mmolar ammonium bicarbonate.

$^1$H NMR (300 MHz, $D_2O$): δ 1.30 (3H, s), 1.38 (3H, s), 3.55 and 3.63 (each 1H, d, J = 15.0 Hz), 4.07 (1H, s), 5.25 and 5.35 (each 1H, d, J = 3.8 Hz), 7.35 (2H, m), 7.71 (2H, m).

The product was subjected to NMR examination with sodium trimethylsilyl propionate-$d_4$ ($1.83 \times 10^{-4}$ mmole) for the determination of β-lactam. The yield of product, 6β-(3-carboxyphenylacetylamino)penicillanic acid, was 68%.

Preparation 10

6β-(3-Carboxyphenylacetylamino)-2α-vinyl-2β-methylpenam-3-carboxylic acid is obtained with the substrate N-(3-carboxyphenylacetyl)-L-cysteinyl-D-γ,δ-didehydroisoleucine and IPNS by the method of Preparation 9.

Preparation 11

6β-(3-Carboxyphenylacetylamino)-2α-methoxy-2β-methylpenam-3-carboxylic acid is obtained by the method of Preparation 9 with the substrate N-(3-carboxyphenylacetyl)-L-cysteinyl-(2R,3R)-2-amino-3-methoxybutanoic acid and IPNS.

III Preparation of 6β-(5-carboxypentanamido)-2α-substituted-2β-methylpenam-3-carboxylic acids via isopenicillin N synthetase.

Preparation 12

Adipoyl-(L)-cysteinyl-(D)-valine

1) Adipic acid mono-benzyl ester

Adipic acid (2.92 g, 20.0 mmol) was dissolved in dimethylformamide (25 ml) and warmed to 70 °C., then dicyclohexylamine (4 ml) was added. After stirring at 70 °C. for 15 minutes, benzyl bromide (3.42 g, 20.0 mmol) was added and the resultant suspension was stirred for a further 20 minutes at 70 °C. and then cooled to 20 °C. Ethyl acetate (100 ml) was added, the suspension was filtered, the filtrate washed with water (3 × 200 ml) and the aqueous layer basified (to pH 11) with aqueous sodium bicarbonate solution. The aqueous layer was separated, washed with ethyl acetate (2 × 100 ml), acidified (to pH 2) with 2N HCl and reextracted with ethyl acetate (2 × 100 ml). The latter organic extracts were combined, washed with water (3 × 100 ml), dried ($Na_2SO_4$), filtered and evaporated to dryness. Chromatography [flash silica (ethyl acetate)] gave 1 as an oil (890 mg, 19%).

TLC (ethyl acetate): $R_f$ 0.8.

$^1$H NMR (60 MHz, $CDCl_3$): δ 1.48-1.83 (2H, m, $CH_2CH_2CH_2CO$), 2.15-2.51 (4H, m, $CH_2CO$), 5.05 (2H, s, $CH_2Ar$), 7.25-7.28 (5H, m, Ar-H), 10.91 (1H, s, $CO_2H$).

m/e (electron impact): 236 ($M^+$, 5%), 91 ($CH_2Ph$, 100%).

2) N-[5-(Benzyloxycarbonyl)pentanoyl]-S-benzyl-L-cysteinyl)-(D)-valine benzyl ester

Reference for coupling procedures:

J.E. Baldwin et al., J. Chem. Soc., Perkin I, 1981, 2253.

1 (787 mg, 3.33 mmol) was coupled with S-benzyl-L-cysteine (using triethylamine/isobutylchloroformate) to give crude N-acylated S-benzyl-L-cysteine (1.53 g, > 100%), which was not purified but coupled with valine benzyl ester (1-ethoxycarbonyl-2-ethoxy-1,2-dihydroquinoline) to give crude 2. Chromatography [flash silica (ethyl acetate/dichloromethane)] gave 2 as an oil (309 mg, 50%).

TLC (10% ethyl acetate/dichloromethane): $R_f$ 0.45.

$^1$H NMR (300 MHz, $CD_3CN$): δ 0.87 (3H, d, J 7 Hz, $CHCH_3$), 0.88 (3H, d, J 7Hz, $CHCH_3$), 1.56-1.61 (4H, m, $CH_2CH_2CH_2CO$), 2.10-2.17 (3H, m, $CH_2CO$, $CHCH_3$), 2.33-2.37 (2H, m, $CH_2CO$), 2.63-2.83 (2H, 8 lines AB part of ABX system $CHCH_2S$), 3.75 (2H, s, $SCH_2Ar$), 4.31-4.36 (1H, m, NCH), 4.51-4.58 (1H, m, NCH), 5.09 (2H, ca s, $CH_2Ar$), 5.11, 5.15 (2H, ABq, J = 12 Hz, $CH_2Ar$), 6.70 (1H, d, J = 7.5 Hz, NH), 7.03 (1H, d,

J = 8 Hz, NH), 7.24-7.37 (15 H, m, Ar-H).

m/e (desorption chemical ionization): 618 ($MH^+$).

Reference for deprotection procedure:

J.E. Baldwin et al., J. Chem. Soc., Perkin I, 1981, 2253.

Liquid ammonia was dried by reflux over sodium, under argon, and was distilled (50-100 ml) into a flask containing a solution of 2 (250 mg, 0.40 mmol) in dry THF (5 ml) at -78° C. Freshly cut sodium was then added in small portions to the stirred reaction mixture at -78° C. until the resultant blue color presisted for 10 minutes. Anhydrous ammonium sulfate was then added until the blue color was discharged, after which the ammonia and THF were evaporated under a stream of argon. The residue was suspended in 50 mmolar aqueous sulfuric acid (20 ml) and the solution was filtered. The precipitate was washed with further 50 mmolar aqueous sulfuric acid (10 ml). Then to the combined filtrate and washings was added Hopkins' reagent (0.40 ml), until precipitation ceased. The precipitate was collected by centrifugation and washed with water (3 × 10 ml). The residual solid was suspended in water and hydrogen sulfide bubbled through the solution for approximately 40 minutes. The black mixture was filtered (celite) and washed with water (10 ml). The filtrate and washings were then combined and evaporated to dryness to give the title compound (85 mg, 60%).

$^1$H NMR (300 MHz, $CD_3OD$): δ 0.90 (3H, d, J = 7 Hz, $CHCH_3$), 0.95 (3H, d, J = 7 Hz, $CHCH_3$), 1.58-1.74 (4H, m, $CH_2CH_2CH_2CO$), 2.08-2.23 (1H, m, $CHCH_3$), 2.24-2.40 (4H, m, $CH_2CO$), 2.77-2.94 (2H, 8 lines AB part of ABX system, $CHCH_2S$), 4.20 [1H, d, J = 5Hz, $CHCH(CH_3)_2$], 4.49-4.58 (1H, m, $CHCH_2$ S).

m/e (fast atom bombardment): 349 ($MH^+$).

Preparation 13

6β-(5-Carboxypentanamido)-2,2-dimethylpenam-3-carboxylic acid

1) General Incubation Procedure for IPNS

An aqueous solution of the substrate (approximately 3 mg) was mixed with aqueous solutions of dithiothreitol (100 mM, 0.100 ml), L-ascorbic acid (50 mM, 0.100 ml), bovine liver catalase (10,000 units/ml, 0.050 ml) and $NH_4HCO_3$ solution (50 mM, 3.5 ml). The pH value was checked and, where necessary, adjusted to 8 by adding dilute NaOH (100 mM). The solution was shaken at 27° C. for 5 minutes and an isopenicillin N synthetase preparation, isolated from *Cephalosporium acremonium* CO 728 (ca 5 I.U./ml, 1 ml) in $NH_4HCO_3$ solution (50 mM) added. The incubation mixture was shaken in two 10 ml vials at 27° C. for 45 minutes, the reaction terminated by precipitating the protein with acetone (7 ml), and the precipitate separated by centrifugation. The crude reaction product was checked by proton NMR (500 MHz) after removal of the acetone *in vacuo* and freeze-drying of the residue.

2) Adipoyl-(L)-cysteinyl)-(D)-valine was incubated with IPNS by following the above general procedure.

Compound isolated by HPLC of the crude incubation mixture:

(2S,5R,6R)-6-(5-carboxypentanamido)-3,3-dimethyl-7-oxo-1-aza-4-thiabicyclo[3.2.0]heptane-2-carboxylic acid

$^1$H NMR(300 MHz, $D_2O$): δ 1.35-1.55 (4H, m, $CH_2CH_2CH_2CO$), 1.36 (3H, s, 3-Me), 1.48 (2H, s, 3-Me), 2.05-2.32 (4H, 2 × m, $CH_2CO$), 4.08 (1H, s, 2-H), and 5.29, 5.39 (2H, 2 × d, J Hz, 5-H, 6-H).

Purification: Reverse phase octadecylsilane HPLC (250 × 4.6 mm column);

Mobile phase: 50 mM $NH_4HCO_3$ (aqueous); Flow rate 1 ml $min^{-1}$;

Retention time = 6.1 min.

Bioassay: positive against *Staphylococcus aureus*.

Alternatively, the freeze-dried incubation mixture was suspended in water (2 ml), acidified (2N HCl) to pH 1, and extracted with ethyl acetate (3 × 5 ml). Excess diazomethane in ether was then added. After stirring for 10 minutes, the solution was evaporated to dryness. Chromatography [preparative layer chromatography (ethyl acetate/dichloromethane)] gave the title compound as an oil.

TLC (ethyl acetate/dichloromethane, 1:1): $R_f$ 0.8.

$^1$H NMR (300 MHz, $CDCl_3$): δ 1.51 [3H, s, $(CH_3)$], 1.58 [3H, s, $(CH_3)$], 1.66-1.74 (4H, m, $CH_2CH_2CH_2CO$), 2.26-2.38 (4H, m, $CH_2CO$), 3.79 (3H, s, $OCH_3$), 4.44 (1H, s, 2-H), 5.55 (1H, d, J = 4 Hz, 5-H), 5.73 (1H, dd, J = 8.56 Hz, 6-H), 6.14 (1H, d, J = 8.5 Hz, 6-H).

m/e (desorption chemical ionization): 373 ($MH^+$, 31%), 200 (15%), 174 (100%).

Preparation 14

Alternate Preparation of 6β-(5-carboxypentanamido)-2,2-dimethylpenam-3-carboxylic acid via 6-APA

1) Adipic acid mono-p-nitrobenzyl ester

The compound 1 was prepared from adipic acid by a procedure identical to that used for the preparation of the mono-benzyl ester above, except p-nitrobenzyl bromide was used instead of benzyl bromide. The yield after recrystallization of the crude material was 0.5 g, 9%, from adipic acid (2.92 g, 20.0 mmol).

TLC (ethyl acetate) $R_f$ 0.5.

$^1$H NMR (60 MHz, $CDCl_3$): δ 1.65-2.04 (4H, m, $CH_2CH_2CH_2CO$), 2.26-2.70 (4H, m, $CH_2CO$), 5.23 (2H, s, $CH_2Ar$), 7.52-8.15 (4H, 2 × ABq, J = 9 Hz, Ar-H), 9.85 (1H, bs, $CO_2H$).

2) 6β-[5-(4-nitrobenzyloxycarbonyl)pentanamido]-2,2-dimethylpenam-4-carboxylic acid 4-nitrobenzyl ester

6-Aminopenicilloic acid p-nitrobenzylester (172 mg, 0.50 mmol) was dissolved in dichloromethane (10 ml) and 1-ethoxycarbonyl-2-ethoxy-dihydroquinoline (EEDQ) (124 mg, 0.50 mmol), compound 1 (140 mg, 0.50 mmol) and anhydrous sodium sulfate (50 mg) were added. The reaction was stirred for 24 hours under an inert atmosphere, after which it was evaporated to dryness. The residue was dissolved in ethyl acetate (50 ml), washed with 2N hydrochloric acid (25 ml), aqueous sodium bicarbonate (25 ml) and brine (25 ml), dried ($Na_2SO_4$), filtered and evaporated to dryness. Chromatography of the residue [flash silica (diethyl ester/dichloromethane)] gave the title product 2).

$^1$H NMR (300 MHz, $CDCl_3$: δ 1.56 (3H, s, $CH_3$), 2.12 (3H, s, $CH_3$), 1.68-1.73 (4H, m, $CH_2CH_2CH_2CO$), 2.26-2.30 (2H, m, $CH_2CO$), 2.41-2.46 (2H, m, $CH_2CO$), 4.49 (1H, s, 2-H), 5.21 (2H, ca s, $CH_2Ar$), 5.28, 5.29 (2H, ABq, J = 13 Hz, $CH_2Ar$), 5.54 (1H, d, J = 4Hz, 5-H), 5.73 (1H, dd, J = 9, 4 Hz, 6-H), 6.10 (1H, d, J = 9 Hz, 6-H), 7.49-7.56 (4H, m, Ar-H), 8.21-8.28 (4H, m, Ar-H).

m/e (electron impact): 614 ($M^+$).

The di-ester product was deesterified to provide the title compound as follows:

To a solution of the diester above (160 mg, 0.17 mmol) in tetrahydrofuran (15 ml) was added a solution of sodium bicarbonate (29 mg, 0.34 mmol) in water (15 ml) and 10% palladium on charcoal (100 mg). The reaction mixture was hydrogenated for 1 hour, after which it was filtered (celite). The filtrate was washed with ethyl acetate (20 ml) and evaporated to dryness to give 5.

$^1$H NMR (300 MHz, $D_2O$) identical to that reported for biosynthetic samples of the title compound.

m/e (fast atom bombardment): 345 ($MH^+$).

## Preparation 15

Alternate Preparation of 6β-(3-carboxyphenylacetylamino)-2,2-dimethylpenam-3-carboxylic acid via 6-APA

1) Isophthalic acid mono p-nitrobenzylester

A mixture of isophthalic acid (3.22 g, 28 mmol), p-nitrobenzyl bromide (6.0 g, 28 mmol) and triethylamine (7.7 ml, 56 mmol) in ethyl acetate (65 ml) was refluxed for 6 hours. Concentration gave a precipitate which was a mixture of 1 and isophthalic acid (ca 1:1) (4.6 g, ca 25%). This mixture was subjected to further transformation without purification.

$^1$H NMR ($CDCl_3$, 60 MHz): δ 5.53 (2H, s, $CH_2C_6H_4NO_2$), 7.42-7.85, 8.08-8.0 and 8.47-8.52 (8H, 3 × m, Ar-H).

2) 3-(p-nitrobenzyloxycarbonyl)phenyl acetic acid

A solution of crude monoester 1 (4 g, ca 50% pure, ca 13 mmol) in benzene (16 ml) was treated with thionyl chloride (16 ml) and heated at 80 °C. for 1.5 hours. After cooling, the solvent was removed in vacuo, benzene (15 ml) was added and again in vacuo in order to remove residual thionyl chloride. The crude acid chloride was redissolved in benzene (20 ml) and treated with an ethereal solution of diazomethane (excess) at 0 °C. and stirred for 20 minutes. The reaction mixture was then evaporated to dryness and treated with p-methoxybenzyl alcohol (6 ml). The resultant mixture was warmed to 50-60 °C. and $Ag_2O$ was added portionwise (3 × 0.3 g) at 20-minute intervals and stirred for a further 30 minutes. The reaction mixture was cooled, diluted with chloroform (30 ml), treated with charcoal (ca 0.5 g), filtered (celite) and evaporated to dryness. Chromatography [flash silica (methanol/dichloromethane)] gave (0.77 g, 10% from isophthalic

acid).

TLC (methanol/dichloromethane, 3:97): $R_f$ 0.5.

m.p.: 148-149 ° C. (from chloroform).

i.r. (KBr disc): 2600-3300 (b), 1728 (s), 1707 (s), 1607 (m), 1522 (m), 1425 (m), 1378 (s), 1351 (m), 1284 (m), 1231 (m), 1198 (m)$cm^{-1}$.

$^1H$ NMR (300 MHz, $CDCl_3$): δ 3.74 (2H, s, Ar$CH_2$$CO_2$), 5.47 (2H, s, $CO_2$$CH_2$Ar), 7.46 (1H, t, J = 9 Hz, Ar-H), 7.51 (1H, d, J = 9 Hz, Ar-H), 7.61 (2H, d, J = 9 Hz, Ar-H), 8.01 (1H, s, Ar-H), 8.02 (1H, d, J = 9 Hz, Ar-H), 8.26 (2H, d, J = 9 Hz, Ar-H).

m/e (desorption chemical ionization): 333 (M + $NH_4^+$), 288, 198 (100%).

| Analysis calculated for $C_{16}H_{13}NO_6$: | | | |
|---|---|---|---|
| Theory: | C, 60.95; | H, 4.61; | N, 4.44 |
| Found: | C, 60.90; | H, 4.11; | N, 4.31 |

3) 6β-[3-(4-Nitrobenzyloxycarbonyl)phenylacetylamino]-2,2-dimethylpenam-3-carboxylic acid 4-nitrobenzyl ester

A solution of (142 mg, 0.45 mmol), 6-aminopenicilloic acid p-nitrobenzyl ester p-toluenesulfonate salt (235 mg, 0.45 mmol), triethylamine (63 µl, 0.45 mmol), and 1-ethoxycarbonyl-2-ethoxy-1,2-dihydroquinoline (EEDQ) (111 mg, 0.45 mmol) in dichloromethane (3 ml) were stirred under an inert atmosphere for 15 hours at 20 ° C. The mixture was diluted with ethyl acetate (50 ml), washed with aqueous sodium bicarbonate (25 ml), brine (25 ml), 10% citric acid solution (25 ml), brine (25 ml), dried ($Na_2SO_4$) and evaporated to dryness. Chromatography [flash silica (ethyl acetate/40-60 petroleum ether)] gave (186 mg, 64%).

i.r. ($CHCl_3$): 3420 (m), 3030 (m), 2970 (w), 2930 (w), 1785 (s), 1750 (s), 1740 (s), 1690 (s), 1610 (s), 1525 (s) $cm^{-1}$.

$^1H$ NMR (300 MHz, $CDCl_3$): δ 1.39, 1.49 (2 × 3H, 2 × s, 2 × $CH_3$), 3.70 (2H, s, Ar$CH_2$CO), 4.45 (1H, s, 2-H), 5.25, 5.30 (2H, ABq, J = 13 Hz, $CH_2$Ar$NO_2$), 5.47 (2H, ca s, $CH_2$Ar$NO_2$), 5.52 (1H, d, J = 4 Hz, 5-H), 5.68 (1H, dd, J = 9, 4 Hz, 6-H), 6.07 (1H, d, J = 9 Hz, NH), 7.45-7.63 (6H, m, Ar-H), 7.99-8.06 (2H, m, Ar-H), 8.20-8.46 (4H, m, Ar-H).

m/e (desorption chemical ionization): 355 (10%), 295 (12%).

$[a]D_D^{20}$ = +99.9 ° (c = 0.96, $CHCl_3$).

The diester was deesterified to yield the title compound as follows:

A solution of (20 mg, 3.1 × $10^{-2}$ mmol) in 50% aqueous tetrahydrofuran (4 ml) containing sodium hydrogen carbonate (5.2 mg, 6.2 × $10^{-2}$ mmol) was hydrogenated over 10% palladium on charcoal (20 mg) for 30 minutes. The reaction mixture was filtered (celite), washed with ethyl acetate (5 ml) and evaporated to dryness to give title compound (11 mg, 84%) as the disodium salt.

$^1H$ NMR, m/e and HPLC retention time were the same as for the compound obtained by natural biosynthetic routes.

Preparation 16

Purification of Deacetoxycephalosporin C Synthetase: Ring Expandase Activity From Acremonium Chrysogenum (Cephalosporium acremonium)

1. Growth of organism

Acremonium chrysogenum strain CO728 was grown in a chemically defined medium. Growth was followed at 550 nm in a Pye Unicam SP6-550 spectrophotometer. Mycelia were harvested and stored at -70 ° C.

2. Preparation of cell-free extracts

These were prepared using a Dyno-Mill grinder (Glen Creston, Stanmore, Middx., G.B.) using a continuous flow method and Tris/HCl buffer (50 mM), pH 7.4, containing DTT (1 mM) and sodium azide (0.015%).

3. Enzyme purification

(a) Initial stages

All steps were carried out at 4°C. Protamine sulfate was added to the cell-free extract to a final concentration of 0.5%. After centrifugation at 12,000 g for 30 minutes, ammonium sulfate was added to the supernatant to a final concentration of 55%. After equilibration and centrifugation (12,000 g for 30 minutes), the supernatant was made up to 75% in ammonium sulfate, and the precipitate collected by centrifugation at 12,000 g for 30 minutes. This material was redissolved in the extraction buffer and loaded onto a Sephadex G-75 gel filtration column (130 × 5 cm), equilibrated with extraction buffer. After elution overnight at 4°C., fractions were tested for expandase activity by bioassay. Active fractions were pooled and applied (flow rate = 24 ml/h) to a column of DEAE-Sepharose Fast Flow (10 × 5 cm), pre-equilibrated with extraction buffer, at 4°C. Enzyme activity was eluted with a linear gradient of 0.05 - 1 M NaCl, over 500 ml, at a flow rate of 24 ml/h. Active fractions, tested by bioassay, were pooled, and the material used for initial studies.

(b) Dye-ligand, ion-exchange FPLC, and gel filtration FPLC chromatography

For purification to near homogeneity, pooled, active fractions after chromatography on a Sephadex G-75 column were applied (flow rate = 10 ml/h) to a column (30 × 2.5 cm) of Procion Red HE3B bound to agarose (Matrex Gel Red A, Amicon Corp., Mass., U.S.A.). In preliminary experiments, procion red (0.1 mM) was found to completely inhibit expandase activity. The column was washed with 350 ml extraction buffer at a flow rate of 24 ml/h, and enzyme eluted with a linear gradient of 0-1.5 M KCl over 500 ml at the same flow rate. Active fractions were pooled and applied (flow rate = 8 ml/min) to a Mono Q 16/10 strong anion-exchange column for FPLC (Pharmacia Ltd.). The enzyme was eluted with a linear gradient of 80-400 mM NaCl in Tris/HCl buffer (20 mM), pH 8.0, containing DTT (2 mM), at the same flow rate. Active fractions were pooled and concentrated and applied (flow rate = 0.25 ml/min) to a Superose 12 FPLC gel filtration column. The enzyme was eluted with Tris/HCl buffer (0.1 M), pH 8.0, containing DTT (2 mM). Active fractions were pooled and concentrated and used in the conversion experiments.

## Examples

General Procedure for Expandase-Substrate Incubation

The expandase was obtained after purification as a suspension in 50 mmolar, pH 7.4, Tris-HCl buffer (ca 0.5-1 I.U./ml). For the incubation of 0.2 mg of the substrate, the following procedure was used. A solution containing ferrous sulfate (0.42 mg, $1.5 \times 10^{-3}$ mmol) and L-ascorbic acid (4.3 mg, $2.4 \times 10^{-2}$ mmol) in water (3 ml) was added to α-ketoglutarate (5.3 mg). The pH of the mixture was then adjusted to pH 7.6 with aqueous sodium hydroxide (100 mmolar). The cofactor solution (0.25 mol) was then added to a solution of the expandase enzyme in 50 mmolar Tris-HCl (1.65 ml, ca 1 I.U.) and the mixture pre-incubated at 270 rpm at 27°C. for 5 minutes. The substrate in 0.1 ml water was then added and the mixture incubated for 2 hours. Protein removal and work-up followed the same procedure as that for incubations using the IPNS enzyme.

### Example 1

7β-(5-Carboxypentanamido)-3-methyl-3-cephem-4-carboxylic acid

6β-(5-Carboxypentanamido)-2,2-dimethylpenam-3-carboxylic acid obtained as described by Preparation 13 was incubated with expandase by following the above general procedure. The compound was isolated by HPLC of the crude incubation mixture.

$^1H$ NMR ($D_2O$, 500 MHz): δ 1.46-1.53 (4H, m, $CH_2CH_2CH_2CH_2$), 1.79 (3H, s, Me), 2.09-2.10, 2.22-2.30 (4H, 2 × m, $CH_2CO$), 3.12, 3.47 (2H, ABq, J=13, 4-H), 5.96, 5.44 (2H, ABq, J=4.5, 6-H, 7-H).

Purification: HPLC details are the same as described in Preparation 13 except: Retention time = 6.8 minutes.

The product was derivatized using the following procedure: the sample of the product as obtained from HPLC was dissolved in 2N HCl (2 ml) and extracted with ethyl acetate (2 × 10 ml). The organic layer was then concentrated to ca 5 ml. Excess diazomethane in ether was then added to the stirred solution. After

stirring for 10 minutes, the solution was evaporated to dryness to give the crude diester.

$^1H$ NMR (500 MHz, $CHCl_3$): δ 1.43-1.71 (4H, m, $CH_2CH_2CH_2CH_2$), 1.70 (3H, s, CMe) 2.29-2.35 (4H, m, 2 × $CH_2CO$), 3.24, 3.50 (2H, ABq, J = 18 Hz, 4-H), 3.68, 3.85 (6H, 2 × s, 2 × OMe), 4.98 (1H, d, J = 4.5 Hz, 6-H), 5.79 (1H, dd, J = 4.5 Hz, 8.5, 7-H), 6.18 (1H, d, J = 8.5 Hz, NH).

m/e ($NH_3$, desorption chemical ionization): 388 (32, M + $NH_4^+$), 371 (34, $M^+$ + 1).

Example 2

7β-(3-Carboxyphenylacetylamino)-3-methyl-3-cephem-4-carboxylic acid

6β-(3-Carboxyphenylacetylamino)-2,2-dimethylpenam-3-carboxylic acid was incubated with expandase by the general procedure.

Compound isolated by HPLC of the crude incubation mixture:

(6R,7R)-1-Aza-3-methyl-7-(m-carboxyphenylacetyl)-8-oxo-5-thiabicyclo[4.2.0]hex-2-ene carboxylic acid.

$^1H$ NMR (500 MHz, $D_2O$): δ 1.77 (3H, 2, $CH_3$), 3.06, 3.37 (2H, ABq, J = 18 Hz, $SCH_2$), 3.57, 3.63 (2H, ABq, J = 15 Hz, $CH_2Ar$), 4.89 and 5.39 (2H, 2 × d, J = 4.5 Hz, 6-H, 7-H), 7.31-7.43 (2H, m, Ar-H), 7.74-7.77 (2H, m, Ar-H).

m/e (fast atom bombardment): 399 ($M^+$ + Na).

Purification: HPLC details are the same as described in Preparation 13 except:

Mobile phase: 2% $CH_3CN$/98% 10 mM $NH_4HCO_3$.

Retention time = 8.5 minutes.

## Claims

## Claims for the following Contracting States : AT, BE, CH, DE, FR, GB, GR, IT, LI, NL, SE

**1.** A process for preparing a compound of the formula

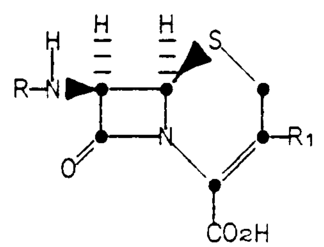

wherein R is 3-carboxyphenylacetyl or adipoyl, and $R_1$ is $C_1$-$C_3$ alkyl, $C_1$-$C_3$ alkoxy, $C_2$-$C_4$ alkenyl or allenyl; which comprises contacting in an aqueous medium at a temperature between about 20 ° C. and about 40 ° C. and at a pH of between about 6 and about 9, a 2β-methyl-2α-substituted penam-3-carboxylic acid of the formula

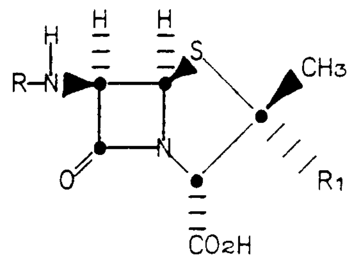

with the enzyme deacetoxycephalosporin C synthetase in the presence of oxygen, ferrous ion, ascorbate, and α-ketoglutarate.

**2.** The process of claim 1, wherein R is 3-carboxyphenyacetyl.

**3.** The process of claim 1, wherein $R_1$ is $C_1$-$C_3$ alkyl, $C_1$-$C_3$ alkoxy, vinyl or allenyl.

**4.** The process of claim 2, wherein $R_1$ is methyl.

**5.** The compound of the formula

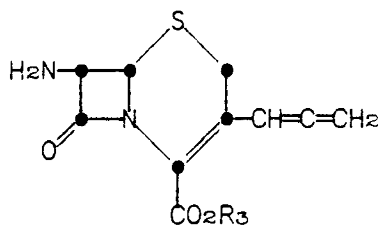

wherein $R_3$ is hydrogen or a carboxy-protecting group.

**6.** The compound of claim 5, wherein $R_3$ is hydrogen, t-butyl, diphenylmethyl or 4-methoxybenzyl.

**Claims for the following Contracting State : ES**

**1.** A process for preparing a compound of the formula

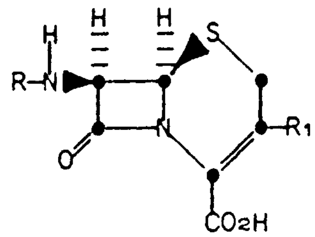

wherein R is 3-carboxyphenylacetyl or adipoyl, and $R_1$ is $C_1$-$C_3$ alkyl, $C_1$-$C_3$ alkoxy, $C_2$-$C_4$ alkenyl or allenyl; which comprises contacting in an aqueous medium at a temperature between about 20° C. and about 40° C. and at a pH of between about 6 and about 9, a 2$\beta$-methyl-2$\alpha$-substituted penam-3-carboxylic acid of the formula

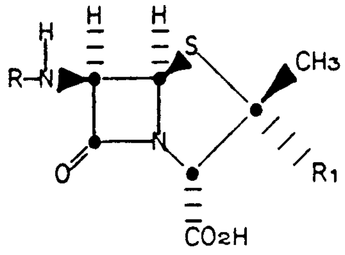

with the enzyme deacetoxycephalosporin C synthetase in the presence of oxygen, ferrous ion, ascorbate, and $\alpha$-ketoglutarate.

**2.** The process of claim 1, wherein R is 3-carboxyphenyacetyl.

**3.** The process of claim 1, wherein $R_1$ is $C_1$-$C_3$ alkyl, $C_1$-$C_3$ alkoxy, vinyl or allenyl.

**4.** The process of claim 2, wherein $R_1$ is methyl.

**Revendications**

**Revendications pour les Etats contractants suivants : AT, BE, CH, DE, FR, GB, GR, IT, LI, NL, SE**

**1.** Procédé pour préparer un composé de formule

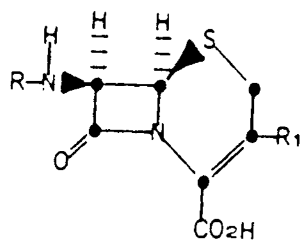

dans laquelle R représente un groupe 3-carboxyphénylacétyle ou un groupe adipoyle et $R_1$ représente un groupe alkyle en $C_1$-$C_3$, un groupe alcoxy en $C_1$-$C_3$, un groupe alcényle en $C_2$-$C_4$ ou un groupe allényle; qui consiste à mettre, dans un milieu aqueux à une température entre environ 20 ° C et environ 40 ° C et à un pH d'environ 6 à environ 9, un acide 2ß-méthyl-2-α-substitué-pénam-3-carboxylique de formule

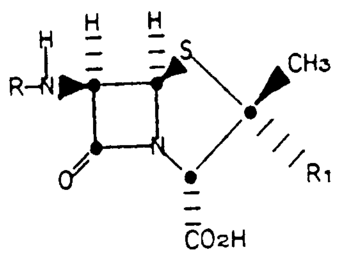

en contact avec l'enzyme désacétoxycéphalosporine C-synthétase en présence d'oxygène, d'ions ferreux, d'ascorbate et d'α-cétoglutarate.

**2.** Procédé selon la revendication 1, dans lequel R représente un groupe 3-carboxyphénylacétyle.

**3.** Procédé selon la revendication 1, dans lequel $R_1$ représente un groupe alkyle en $C_1$-$C_3$, un groupe alcoxy en $C_1$-$C_3$, un groupe vinyle ou un groupe allényle.

**4.** Procédé selon la revendication 2, dans lequel $R_1$ représente un groupe méthyle.

**5.** Composé de formule

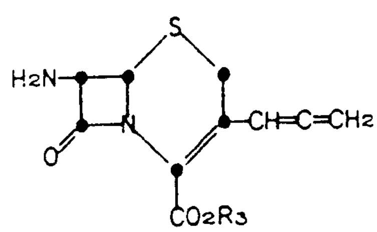

dans laquelle $R_3$ représente un atome d'hydrogène ou un groupe protecteur du groupe carboxyle.

**6.** Composé selon la revendication 5, dans lequel $R_3$ représente un atome d'hydrogène, un groupe t-butyle, un groupe diphénylméthyle ou un groupe 4-méthoxybenzyle.

**Revendications pour l'Etat contractant suivant : ES**

**1.** Procédé pour préparer un composé de formule

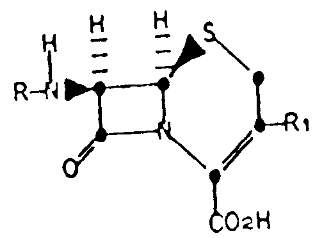

dans laquelle R représente un groupe 3-carboxyphénylacétyle ou un groupe adipoyle et $R_1$ représente un groupe alkyle en $C_1$-$C_3$, un groupe alcoxy en $C_1$-$C_3$, un groupe alcényle en $C_2$-$C_4$ ou un groupe allényle; qui consiste à mettre, dans un milieu aqueux à une température entre environ 20 ° C et environ 40 ° C et à un pH d'environ 6 à environ 9, un acide 2ß-méthyl-2-α-substitué-pénam-3-carboxylique de formule

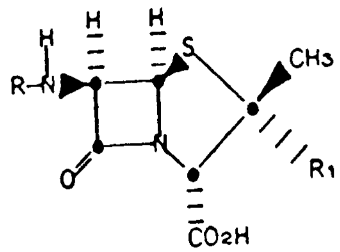

en contact avec l'enzyme désacétoxycéphalosporine C-synthétase en présence d'oxygène, d'ions ferreux, d'ascorbate et d'α-cétoglutarate.

**2.** Procédé selon la revendication 1, dans lequel R représente un groupe 3-carboxyphénylacétyle.

**3.** Procédé selon la revendication 1, dans lequel $R_1$ représente un groupe alkyle en $C_1$-$C_3$, un groupe alcoxy en $C_1$-$C_3$, un groupe vinyle ou un groupe allényle.

**4.** Procédé selon la revendication 2, dans lequel $R_1$ représente un groupe méthyle.

**Patentansprüche**
**Patentansprüche für folgende Vertragsstaaten : AT, BE, CH, DE, FR, GB, GR, IT, LI, NL, SE**

**1.** Verfahren zur Herstellung einer Verbindung der Formel

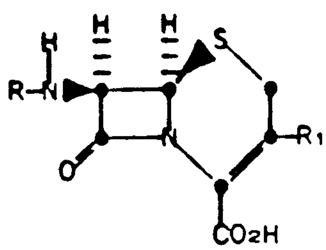

worin R ein 3-Carboxyphenylacetyl- oder Adipoylrest ist und $R_1$ ein $C_1$-$C_3$-Alkyl-, $C_1$-$C_3$-Alkoxy-, $C_2$-$C_4$-Alkenyl- oder Allenylrest ist; umfassend, daß man in einem wäßrigen Medium bei einer Temperatur zwischen etwa 20 ° C und etwa 40 ° C bei einem pH zwischen etwa 6 und etwa 9 eine 2ß-Methyl-2α-substituierte Penam-3-carbonsäure der Formel

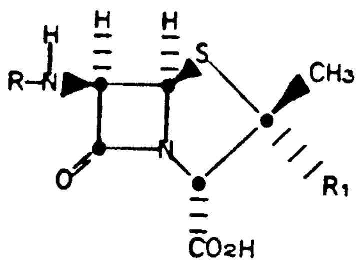

mit dem Enzym Deacetoxycephalosporin-C-Synthetase in Gegenwart von Sauerstoff, Eisen-II-ion, Ascorbat und α-Ketoglutarat in Kontakt bringt.

2. Verfahren nach Anspruch 1, worin R ein 3-Carboxyphenylacetylrest ist.

3. Verfahren nach Anspruch 1, worin $R_1$ ein $C_1$-$C_3$-Alkyl-, $C_1$-$C_3$-Alkoxy-, Vinyl- oder Allenylrest ist.

4. Verfahren nach Anspruch 2, worin $R_1$ ein Methylrest ist.

5. Verbindung der Formel

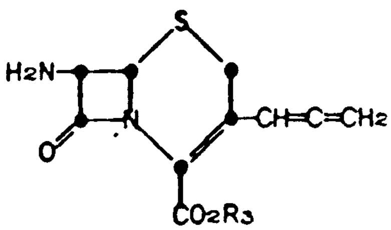

worin $R_3$ Wasserstoff oder eine Carboxyschutzgruppe ist.

6. Verbindung nach Anspruch 5, worin $R_3$ Wasserstoff, ein t-Butyl-, Diphenylmethyl- oder 4-Methoxybenzylrest ist.

**Patentansprüche für folgenden Vertragsstaat : ES**

1. Verfahren zur Herstellung einer Verbindung der Formel

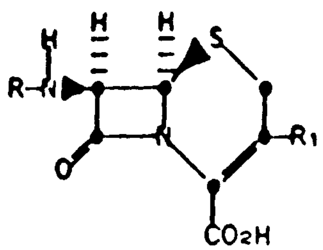

worin R ein 3-Carboxyphenylacetyl- oder Adipoylrest ist und $R_1$ ein $C_1$-$C_3$-Alkyl-, $C_1$-$C_3$-Alkoxy-, $C_2$-$C_4$-Alkenyl- oder Allenylrest ist; umfassend, daß man in einem wäßrigen Medium bei einer Temperatur zwischen etwa 20°C und etwa 40°C bei einem pH zwischen etwa 6 und etwa 9 eine 2ß-Methyl-2α-substituierte Penam-3-carbonsäure der Formel

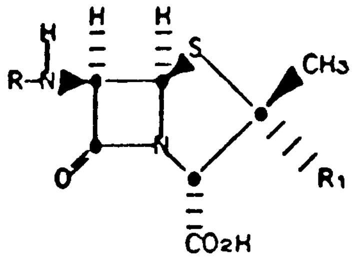

mit dem Enzym Deacetoxycephalosporin-C-Synthetase in Gegenwart von Sauerstoff, Eisen-II-ion, Ascorbat und α-Ketoglutarat in Kontakt bringt.

2. Verfahren nach Anspruch 1, worin R ein 3-Carboxyphenylacetylrest ist.

3. Verfahren nach Anspruch 1, worin $R_1$ ein $C_1$-$C_3$-Alkyl-, $C_1$-$C_3$-Alkoxy-, Vinyl- oder Allenylrest ist.

4. Verfahren nach Anspruch 2, worin $R_1$ ein Methylrest ist.